# EUROPEAN PATENT SPECIFICATION

(11) **EP 2 231 171 B1**
(45) Date of publication and mention of the grant of the patent: **05.10.2016**
(21) Application number: 08861633.9
(22) Date of filing: 12.12.2008
(51) Int. Cl.: A61K 33/26, A61K 38/17, A61P 7/06

(54) **COMPOSITIONS AND METHODS FOR INCREASING IRON ABSORPTION**
ZUSAMMENSETZUNGEN UND VERFAHREN ZUR ERHÖHUNG DER EISENABSORPTION
COMPOSITIONS ET MÉTHODES ACCROISSANT L'ABSORPTION DE FER

(30) Priority: 14.12.2007 US 13873 P
(43) Date of publication of application: 29.09.2010
(73) Proprietor: Hofseth Biocare ASA, 6004 Ålesund (NO)
(72) Inventor: FRAMROZE, Bomi, Toronto ON M5P 1T6 (CA)
(74) Representative: Zacco Norway AS
(86) International application number: PCT/US2008/086668
(87) International publication number: WO 2009/079401

(56) References cited:
- JP-A- 4 158 761
- JP-A- 5 229 959
- US-A- 5 662 922
- US-A1- 2004 087 504
- US-A1- 2007 087 039
- DATABASE WPI Week 200531 Thomson Scientific, London, GB; AN 2005-296923 XP002621938, & CN 1 561 840 A (HUANGHAI AQUATIC PROD INST CHINA AQUATIC) 12 January 2005 (2005-01-12)

## Description

### FIELD

The present invention relates to increased iron absorption. The present invention also relates to compositions comprising iron and peptides obtained by enzymatic hydrolysis. The present invention also relates to causing a human or an animal to ingest peptides obtained from enzymatically hydrolyzed marine vertebrate.

### BACKGROUND

Iron is essential to human and animal physiology. Insufficient iron in humans and animals can cause a number of diseases and disorders, including iron deficient anemia and stunted growth.

Current practice for providing additional iron to animals is via injection.

For humans, iron supplements are available in pill form.

JP 5229959 discloses an iron absorption accelerating composition comprising ESP-2 and DAN-1 obtained from sardine muscle, rather than peptides used in the composition of the present invention which are derived from the backbone and head of a salmon.

### SUMMARY

The invention provides:
1. A composition for use in increasing absorption of iron when ingested by an animal or human, said composition comprising iron and peptides, wherein:
   (a) the peptides are obtained from enzymatically hydrolyzed salmon backbones and heads;
   (b) the composition is suitable for human or animal ingestion; and
   (c) the peptides are greater than 50% of the composition.
2. The composition for use of 1, wherein the molecular weights of the peptides are 3500 Daltons or less.
3. The composition for use of 1 or 2, wherein the iron is greater than 0.01 % of the composition.
4. The composition for use of any of 1 to 3, further comprising fish oil.
5. The composition for use of any of 1 to 4, wherein the composition is a dietary supplement of feed additive.
6. The composition for use of any of 1 to 5, wherein less than 50% of the peptides are chelated to the iron.
   The animal is a mammal or bird.
7. The composition of any of 1 to 6 for use in treatingan animal or human which has iron deficient anemia or is at risk for having iron deficient anemia and does not have a diet that is altered because of a disease or disorder.

### DETAILED DESCRIPTION

Provided, in an aspect of the invention, are compositions for increasing iron absorption comprising peptides obtained from enzymatically hydroiyzed fish protein.

As used herein, the term "increasing iron absorption" has its common meaning in the art. Accordingly, "increasing iron absorption" includes increasing the intestinal absorption of iron into the blood stream. As a further example, the term includes increasing uptake or storage of iron in cells or in transport proteins. Increased iron absorption can be measured by a variety of means known to the skilled artisan. For instance, iron absorption can be measured by assaying levels of transferrin or ferritin.

As used herein, the term "iron" has its common meaning in the art in the context of iron that is ingested by humans or animals. Accordingly, the term includes heme iron, non-heme iron, elemental iron (also known as reduced iron), complexed iron, chelated iron, iron salts and other forms of iron that are suitable for human or animal ingestion. Iron for ingestion may be obtained from numerous sources known to the skilled artisan. For example, iron for ingestion can be obtained from animal food sources, such as red meat, fish or poultry. Such iron is commonly referred to as heme iron. Iron for ingestion may also be obtained from plant food sources, such as vegetables and fruits. Such iron is commonly referred to as non-heme iron. Iron for ingestion may also be obtained from microbial sources, for example, from Green Algae.

Iron for ingestion may also be obtained from non-organic sources. For example, ferrous or ferric iron salts are a source of ingestible iron, for instance, ferrous sulfate, ferrous fumarate, ferrous succinate, ferrous gluconate, ferrous lactate, ferrous glutamate, and ferrous glycine. Also, elemental iron powder can be used as a source of ingestible iron. Additionally, iron may be obtained from the same sources as the peptides described herein. For example, the iron and peptides may both be obtained from fish, for example, from North Atlantic Salmon (e.g. *Salmon salar* and other fish from this genus).

In an aspect of the invention, the iron in the compositions is greater than 0.01% of the composition. For example, in the case of a composition having a total dry weight of 1 kilogram, the dry weight of iron would be greater than 100 milligrams. In a further aspect of the invention, the percentage of iron is greater than 10% of the total composition. In a further aspect of the invention, the percentage of iron in the composition is between 0.01 % to 10% of the total composition and in a further aspect, the percentage of iron is greater than 0.05%, greater than 0.1 % and greater than 1, 2, 3, 4, 5, 6, 7, 8 or 9% of the total composition.

As used herein, the term "peptides" has its common meaning in the art.
Accordingly, peptides include dimeric peptides, multimeric peptides and polypeptides. In an aspect of the invention, the molecular weights of the peptides are less than 60,000 Daltons. In a further aspect of the invention, 100% or at least 90% of the peptides have a molecular weight of less than 10,000 Daltons. In another aspect of the invention, at least 70% of the peptides have a molecular weight of less than 5,000 Daltons. In another aspect of the invention, at least 50% of the peptides used have a molecular weight of less than 1,000 Daltons. In another aspect of the invention, 100% of the peptides have a molecular weight of 3500 Daltons or less.

In an aspect of the invention, the amount of peptides in the compositions is greater than 50% of the composition. In another aspect of the invention, the amount of peptides is greater than 75%, greater than 90% or greater than 99% of the composition. Alternatively, in another aspect of the invention, the amount of peptides is greater than 50-99% of the composition or greater than any integer percentage between 50-99%. Accordingly, as a non-limiting example, an aspect of the invention includes a composition having a total dry weight of 1 kilogram in which the total dry weight of the peptides in the composition is greater than 500 grams, that is, greater than 50% of the total dry weight of the composition. In another aspect of the invention, the percentage is based on caloric amount. According, as a further non-limiting example, an aspect of the invention includes a composition having a total caloric content of 1000 calories in which the caloric content of the peptides is greater than 500 calories, that is, greater than 50% of the total caloric content of the composition.

As used herein, the term enzymatic hydrolysis of proteins has its ordinary meaning of the art. Accordingly, the invention includes peptides resulting from partial hydrolysis.

Referring to the enzymatic hydrolysis aspects of the invention, several protein hydrolysis enzymes (also knows in the art as proteolytic enzymes or proteases) are available to the skilled artisan. For example, endopeptidases and/or exopeptidases may be used. Additionally, the following enzymes or combinations of the following enzymes may be used: Alcalase^{®}, Neutrase^{®}, Protamex^{®}, Flavourzyme^{®} (each available from Novozymes), Pescalase^{®}. (available from Gist- brocades of the Netherlands) and Promo 31^{®} (available from Biocatalysts Ltd. of Wales). An example of a combination of enzymes that may be used is a mix of two enzymes Alcalase^{®} and Neutrase^{®} in a ratio from 1:9 to 1:1.

Further methods of enzymatic hydrolysis are known in the art.
Furthermore, descriptions of enzymatic hydrolysis and fish amino acids obtained by enzymatic hydrolysis are found, for example, in U.S. published patent application, publication number: US 2005/0037109 A1 to Soerensen et al.

In additional to peptides and iron, the inventive compositions s may also include compounds, such as vitamins, minerals, non-protein nitrogen, carbohydrates or other compounds associated with enzymatic hydrolysis.

In a further aspect of the invention, compositions include lipids in addition to the peptides and iron. For examples, fish oil may be used in combination with peptides in order to increase iron absorption by humans and animals. In a further aspect of the invention, fish oil obtained from the same source as the fish peptides may be used. In another aspect of the invention, however, oils with high levels of oxidized fatty acids are avoided because fatty acid oxidation in general will decrease palatability. In a further aspect of the invention, the level of unsaturation in the lipids used is below a pre-determined amount.

In a further aspect of the invention, iron or the peptides, or the two in combination, are dietary supplements or feed additives. The term "dietary supplement" or "feed additive" refers to the common meaning these terms have in the art. Accordingly, a dietary supplement or feed additive means that the peptides or iron are obtained from a source that is separate from an animal or human's diet Moreover, a dietary supplement or feed additive is often processed differently or separately from food or feed and is often packaged in separate containers from food or feed. Dietary supplements or feed additives may also have a distinct chemical composition. For example, iron as a dietary supplement or feed additive is commonly in the form of ferrous sulfate as compared to iron found in food or feed which is commonly in the form of meat heme iron. Prescription pet food (for a companion animal) can be formulated according to the subject invention. Such prescription food is typically for pets of advanced age whose nutritional uptake becomes problematic.

Aside from humans, particularly those suffering from or at risk for situational anemia (where nutritional challenges exist, for example), other animals can be provided with compositions according to the subject invention. Such animals include production animals such as pigs {and other swine), chickens (and poultry), cows and cattle (bovines), and other such animals. Of particular interest are animals having or at risk of developing situational anemia, such as weaning piglets and other growing or developing animals such as those listed above (calves, for example). Formulation of the subject invention can be palatable to such animals even where fish is not part of their natural diet (as opposed to minks, for example, which naturally eat fish).

In a further aspect of the compositions of the invention, less than 50% of the iron is chelated to peptides. In a further aspect, the percentage of iron peptide chelation is less than 50% to 0% and in another aspect less than any integer percentage between 50% and 1%.

With regard to the inventive composition, amounts may be calculated in several alternative ways, for example, weight/volume (VWV), volume/weight (VΛ/V), volume/volume (V/V), or weight/weight (W/W). Furthermore, amounts can be calculated based on caloric percentages. As used herein, amounts and percentages include such amounts and percentages measured by any methodology that would be reasonably applied by the person of skill in the art. For example, a composition having greater than 50% peptides, as set forth herein, includes compositions having greater than 50% peptides based on dry weight of peptides to dry weight of total composition as well as compositions having 50% peptides based on other calculation methodologies known to the skilled artisan, such as, weight of peptides to volume of total composition (W/V) in the case of a liquid composition. Accordingly, in another aspect of the invention, the amount of peptides, measured by any of above mentioned methods of calculations, is greater than 50%. The foregoing applies to all amounts and percentages set forth herein, including amounts and percentages of peptides, iron and chelated peptides.

In another aspect of the invention, the inventive compositions do not contain significant amounts of compounds, such as trimethylaminoxide, that induce the formation of crystalline, insoluble iron oxide hydrates, that may inhibit iron absorption.

The compositions of the invention are directed to increasing the absorption of iron in an animal or human. In an aspect of invention, an animal or human is caused to ingest the peptides. In a further aspect of the invention, the peptides are ingested together or separately from iron. In another aspect, iron is ingested within a reasonable time before or after peptide ingestion. In a further aspect, the iron is ingested less than 24 hours before or after the peptides are ingested. In a further aspect of the invention, the iron is ingested less than 24 hours to less than 1 hour (and any range of hours in between) before or after the peptides are ingested.

In an aspect of the invention, the inventive compositions ameliorate diseases and disorders resulting from inadequate iron in a human or animal. Perhaps the most common of such disorders is iron deficient anemia. Anemia is a disorder characterized by a deficiency of red blood cells. Iron deficient anemia is anemia that is caused by an inadequate amount of iron. This iron deficiency may result from inadequate amounts of iron ingested, inadequate absorption of iron, blood loss or a combination of these.

Iron deficient anemia is also common in animals or humans during periods of growth, such as infancy or weaning. Iron deficient anemia is also found in livestock bred to have a high growth to feed ratio.

Anemia by iron deficiency can be distinguished from many other anemias. For example, iron deficient anemia is different from anemia resulting from chronic infectious, inflammatory, or malignant disorders, such as arthritis or cancer. More specifically, the following anemias are distinct from iron deficiency anemia: hypochromic anemia, microcytic anemia, chlorosis, hereditary sideroblastic anemia, idiopathic acquired sideroblastic anemia, red cell aplasia, megaloblastic anemia, such as pernicious anemia, vitamin B12 deficiency and folic acid deficiency anemia, aplastic anemia, hemolytic anemias, such as autoimmune helolytic anemia, microangiopathic hemolytic anemia, and paroxysmal nocturnal hemoglobinuria.

Moreover, in another aspect of the invention, iron deficient anemia does not include anemia resulting from kidney failure, decreased kidney function or dialysis.

As mentioned herein, the peptides ingested increase absorption of the iron in the animal or human. In a further aspect of the invention, the peptides ingested substantially increase absorption of iron in the human or animal. In further aspects of the invention, iron absorption is increased by a statistically significant amount and in a further aspect iron absorption is increased by 50% up to greater than 100%. In a further aspect, iron absorption is increased by a percent that is greater than any integer percentage between 50% and 100%. As mentioned above, an increase in iron absorption can be assessed by measuring transferrin or ferritin or by other methods known to the skilled artisan.

Referring to other aspects of the inventive compositions, the term "ingest" has its common meaning in the art. Typically ingestion is made by mouth. However, other means of ingestion, such as a naso-gastric tube, may also be used. Moreover, in addition to ingestion, in a further aspect of the invention, iron supplements may be administered intravenously.

In a further aspect of the invention, ingestion is by an animal or human on a normal protein diet. The term "normal" here has its ordinary meaning in the art in the context of normal versus a diet that is altered because of a disease or disorder. For example, an animal or human suffering from a kidney dysfunction often will not have a normal protein diet because such a subject cannot properly metabolize proteins.

The phrase "suitable for human or animal ingestion" refers to the common meaning of this phrase in the art. For example, "suitable for human or animal ingestion" refers to an acceptable amount, for human or animal safety, of pathogens and toxins (in the case of toxins, for example, mercury and other heavy metals).

For instance, it is known to the skilled artisan, that levels of certain pathogens should be below a predetermined amount for a composition to be suitable for human or animal ingestion. For example, in the context of pathogenic microorganism, and in an aspect of the invention, the amount of certain pathogens (as known to the skilled artisan) should be below a Minimum Infective Dose (MID). MID refers to the minimum number of pathogenic microorganisms capable of causing a disease. For example, the MID for *Vibrio cholerae,* a pathogenic microbial contaminant found in fish derived food, is 10⁶. Accordingly, in an aspect of the invention, the compositions herein contain *Vibrio cholerae* below this MID.

In addition to *Vibrio cholerae,* the following pathogens are known to the skilled artisan and in an aspect of the invention, the compositions contain amounts of these pathogens below the respective pathogen MIDs: *Salmonella spp., Clostridium botulinum, Staphylococcus aureus, Yersinia enterocolitica, Yersinia pseudotuberculosis, Listeria monocytogenes,* other *Vibrios* strains, and various *E coli* strains.

Similarly, in another aspect of the invention, the compositions contain level of pathogen viruses, protozoa and worms so that the compositions are considered suitable for human or animal ingestion.

Moreover, methods for achieving desired reduced levels of pathogens are known to the skilled artisan. For example, cooking, chilling, irradiation (for example by X-rays or gamma rays), heating, pressure heating, freezing or a combination of these are known to the skilled artisan to be safe and effective processes for reducing pathogen levels of compositions to be ingested by animals or humans.

As another example, it is known to the skilled artisan that reducing the size (molecular weight) of peptides in a composition is a common way to minimize the risk of prion infection. Accordingly, in an aspect of the invention, the peptides have the molecular weights set forth above.

The above description and the below examples do not limit the compositions described herein and only serve to illustrate various non-limiting embodiments.

Moreover, herein, the use of the singular is not meant to be limiting.
For example, the description of "a composition" or "the composition" is not meant to limit the invention to a single composition. That is, it is understood that a composition herein, when combined with other compositions, is still within the scope of the present invention, unless otherwise explicitly set forth herein.

Additionally, the following words should be read as follows unless another meaning is explicitly set forth. The words "include," "includes" or "including" means to include, without limitation. The words "comprise," "comprises" or "comprising" mean to include without limitation. The word "or" means and/or.

### EXAMPLES

### For Examples 1 and 2, blood was collected from six dogs fed 20%

Salmon Protein Hydrolysate (SPH), six dogs fed 20% Salmon Protein Hydrolysate and 4.5% weight/weight (W/W) Salmon Oil (SPHO) and six control dogs. All dogs were fed the control diet which consisted of 30% poultry by-product protein, and 25% fat, from day one to day fourteen. The non-control dogs were fed the SPH and SPHO from day fifteen through day twenty-six only. The six control dogs were fed the control diet without the above feed additives.

Examples 1 and 2 focus on physiological effect studies on a series of in vitro assays. The examples find enzymatic marker based changes in plasma concentration. The two in vitro assays together investigate potential presence of bioactive peptides of the SPH and SPHO feeds that impact storage iron concentration in the feed subjects. The two in vitro assays address transferrin and ferritin. The first assay (Example 1) addresses transferrin, the second assay (Example 2) addresses ferritin.

### EXAMPLE 1

The serum transferrin levels of the dogs were measured. The assay used to measure transferrin levels was an ELISA assay. This test is a two site enzyme linked immunoassay for measuring transferrin in serum or plasma of dogs. Transferrin serum measurement is of particular value in identifying iron absorption efficiency, especially when coupled with the simultaneous measurement of plasma ferritin levels.

Transferrin is a protein that binds to iron. Transferrin functions to transport iron to the bone marrow and to tissue storage organs such as the liver. Transferrin also participates in the regulation and control of iron absorption and protects against iron intoxication.

The transferrin assays results are summarized in Tables 1 and 2.

**TABLE 1: Corrected Average Transferrin Concentration (mg/L)**

| Dog | 1 | 2 | 3 | 4 | 5 | 6 |
|---|---|---|---|---|---|---|
| SPHO | 1.642 | 1.874 | 1.480 | 1.503 | 1.317 | 1.341 |
| SPH | 2.570 | 2.686 | 2.686 | 2.593 | 2.338 | 2.199 |
| Control | 2.988 | 4.449 | 4.148 | 3.591 | 3.243 | 2.709 |

**TABLE 2: Corrected Average Transferrin Concentration (mg/L)**

| | Standard Deviation | Mean | Standard Error of the Mean |
|---|---|---|---|
| SPHO | 0.223 | 1.526 | 0.091 |
| SPH | 0.221 | 2.512 | 0.090 |
| Control | 0.696 | 3.023 | 2.84 |

### EXAMPLE 2

Ferritin is an iron binding protein involved in iron storage. Ferritin is located in various tissues, for example, liver, spleen, bone marrow and mucus of the bowels.

The ferritin assay results are summarized in Tables 3 and 4.

**TABLE 3: Corrected Average Ferritin Concentration (mg/L)**

| Dog | 1 | 2 | 3 | 4 | 5 | 6 |
|---|---|---|---|---|---|---|
| SPHO | 277.543 | 267.799 | 246.920 | 324.173 | 335.308 | 271.279 |
| SPH | 221.169 | 147.397 | 248.312 | 230.217 | 291.462 | 270.583 |
| Control | 113.294 | 131.389 | 265.711 | 298.422 | 146.701 | 141.829 |

**TABLE 4: Corrected Average Ferritin Concentration (mg/L)**

| | Standard Deviation | Mean | Standard Error of the Mean |
|---|---|---|---|
| SPHO | 38.457 | 287.171 | 15.700 |
| SPH | 55.441 | 234.857 | 22.634 |
| Control | 78.877 | 164.009 | 32.201 |

### Discussion of Results

The use transferrin and ferritin concentrations to assure the impact of
SPH and SPHO iron absorption. The results show that SPHO and SPH increased plasma concentrations of ferritin and decreased plasma concentrations of transferrin. The ferritin increase suggests increased iron absorption and/or bioavailability. Similarly the transferrin concentration decrease suggests a reduction in iron transport. The transferrin concentration decrease also suggests a reduced need for iron absorption due to (it is hypothesized) increased iron bioavailability and/or increased concentrations of ferritin bound iron. The transferrin concentration decrease and the ferritin concentration increase support a conclusion that iron, absorption and bioavailability were increased in the dogs fed SPHO and SPH in relation to the control dogs.

The transferrin concentration decrease showed statistical significance.
The ferritin concentration increase showed a trend, but was technically not statistically significant due to scatter in individual concentration levels across all three groups of dogs.

### EXAMPLE 3

### Caco-2 Cell Iron Uptake measurements using SP Dry PP1 in comparison with Meat, Chicken, Fish, and Casein Protein digests

Salmon backbones and heads, separated after filleting of whole salmon, were subjected to protein hydrolysis using a manufacturing process developed by Green Earth Industries (GEI). See e.g. US 2005/0037109. This process yields a water soluble protein fraction (SP), an insoluble protein fraction (NSP), a oil fraction (FO) and a bones fraction (CA). These fractions can be separated from each other with particular attention to overall quality and separations for the SP fraction. The SP is first isolated from the process as a 6-7% solution, which is concentrated to a 55-60% suspension in a triple effect falling film evaporator. This honey-like material is further dried, using spray-drying equipment, to yieid a pale yellow protein powder (SP Dry).

SP Dry was fed to senior dogs in a digestibility and palatability study by
GEI, and the plasma was collected and analyzed. It showed a significant increase in ferritin and decrease in transferrin proteins, which is indicative of increased iron uptake in the animals receiving the test SP Dry and SP Dry + Salmon oil fraction.

Iron absorption by animal models (such as dogs and guinea pigs) offer direct and physiologically relevant assessment of iron bioavailability. We also demonstrated the iron uptake potential of "SP Dry" using a Caco-2 cell iron uptake in-vitro method (Iron availability from Iron-Fortified Sprulina by an in-vitro digestion/Caco-2 Cell Culture Model. Jean-Max Rouanet et al. Journal of Agric, Food Chem. 49(3), 1625, (2001) to rapidly estimate the potential increase in iron uptake via the incorporation of SP Dry in the diet.

This study was undertaken to analyze the performance of SP Dry to potentiate iron uptake, in comparison with casein, soy, meat, chicken and fish proteins after a simulated peptic and intestinal digestion process.

This Caco-2 cell study combines simulated peptic and intestinal digestion and iron uptake measurement by a Caco-2 cell monolayer.

The SP Dry PP1 sample of soluble protein hydrolysate was produced under the supervision of Meatzyme B.V. and Green Earth LLC. The soluble protein fraction was spray dried and received in Mumbai, India in good condition for chemical analysis. The SP Dry PP1 sample is a pale yellow amorphous powder with a mild fish odor.

The meat (beef), chicken (thigh) and fish (tuna fillet) protein samples were purchased fresh and treated as follows. All visible fat and connective tissue was removed and sliced into thin slices. After simmering in deionized water for 30 minutes, the pieces were removed, chilled at 0°C and further visible fat was removed. The pieces were homogenized in a blender with minimum water, lyophilized and passed through a 40 mesh sieve to give the protein powders used herein. The casein was purchased from the Parsi Dairy Farm Ltd. and used as is.

The in-vitro digestion of the above four protein sources was carried out using porcine pepsin and bile extract (for simulating stomach digestion) and pancreatin and biie extract (for simulating intestinal digestion) purchased from Sigma Aldrich and used as is in a standard digestion procedure. ⁵⁹Fe labeled iron (III) chloride was used to make up the iron concentrations to 10 µmol/L in the homogenates.

Initial protein content was measured after solubilizing in 0.5 mol/L sodium hydroxide and using the Bio-Rad^{®} DC protein assay kit.

Digested protein content was measured as the TCA solubilized precipitate using the same kit, wherein 20 ml of each digest was diluted to 50 ml with deionized water and reacted with 50 ml of 0.6 mol/L trichloroacetic acid. The samples were centrifuged and the precipitate solubilized in 0.5 mol/L and measured as above.

A modified form of the commercially available 24x well Caco-2 assay kit from Celsis In-vitro Technologies was used in this assay. The Celsis kit was pre-plated with Caco-2 cells with Corning Costar Transwell@ filters. The IVT Caco-2 cultures are considered acceptable for transport studies and meet the transepithelial electrical resistance (TEER) criteria of 1000 ohms.

1.5 ml of each post-digestion protein solution containing 10µmol/L of ⁵⁹Fe was added to 12 wells each, containing normal Caco-2 cells in the lower chamber and the plates incubated at 37°C for two hours before separating the layers.

The cells were extracted using the procedure described by Celsis, and ⁵⁹Fe was counted using an automated gamma counter and determined as the fraction remaining in the supernatant after centrifuging.

Initial content parameters (before ⁵⁹Fe addition) and the estimated percent digestion as measured by TCA precipitable protein measurement are shown in Table 5 below.

**TABLE 5 - Analysis of the proteins homogenates before and after digestion**

| Source | % Protein | Crude fat (mg/g) | % moisture | Total Iron | % Digestion |
|---|---|---|---|---|---|
| Meat | 84% ±2% | 110 ± 5 | 6.1 ± 0.2 | 0.84 ± 0.01 | 66% ± 5% |
| Chicken | 88% ± 1% | 90 ± 5 | 4.9 ± 0.2 | 0.65 ± 0.01 | 68% ± 5% |
| Fish | 94%±1% | 40 ± 2 | 6.1 ±0.2 | 0.13 ± 0.01 | 74% ± 5% |
| Casein | 92% ± 1% | ND | 5.8 ± 0.2 | 0.03 ± 0.01 | 88% ± 5% |
| SP Dry | 93% ± 0.5 % | 36 ± 2 | 4.2 ± 0.2 | 0.007 ± 0.001 | 93%±1% |

* Total Iron was measured on a spectrometer as a Fe-ferene complex at 595 nm (Ferene - a new spectrophotometric reagent for iron. D. D. Hennessy et al. Can. J. Chem. 62, P721, (1984).

The results of the iron uptake variation between different protein sources, is shown in Table 6 below. The muscle protein sources (meat, chicken and fish) showed similar iron uptakes in the Caco-2 cells below the membrane, while the casein showed almost no uptake, which was very similar to the negative (osmosis) control buffer solution. The SP Dry product showed a significantly higher iron uptake as compared to all the other protein sources.

**TABLE 6- Cell uptake of iron (nmol/mg cell protein, after 2 hrs equilibration)**

| Buffer Solution | Meat | Chicken | Fish | Casein | SP Dry |
|---|---|---|---|---|---|
| 0.015 ± 0.005 | 0.095 ± 0.005 | 0.070 ± 0.005 | 0.085 ± 0.005 | 0.025 ± 0.005 | 0.135 ± 0.005 |

The Caco-2 model has been successfully used to study drug uptake in dozens of experiments in the literature. (Intestinal absorption of ⁵⁹Fe from neutron activated commercial oral iron (III) citrate and iron (III) hydroxide polymaltose complexes in man. (H. C. Heinrich Arzneim-Forsch/Drug.Res. 37, 105, (1987).) The modified assay used here has also been used to study fortification of food stuff not only with iron but other essential elements such as zinc and vitamin-D. This in-vitro model has repeatedly shown excellent comparability with human absorption in the intestine and hence the results shown in this study support the positive results from the Fahey senior dog feeding plasma study. An animal model study can also be conducted using guinea-pigs, for example, to further study the effect of feeding SP Dry on RBC (hemoglobin).

It is apparent from this study that the marine protein digest - SP Dry - shows a positive effect on iron uptake as compared to the other digested protein sources tested. SP Dry improved iron uptake by 540% over the negative control casein and was even 40%+ higher than the positive control, meat protein digest. Salmon Oil (SO) was not used in this experiment at all, and hence this is conclusive evidence that the protein fraction by itself potentiates an increase in iron uptake.

It should also be noted that only the extrinsic added iron (10mmol/L) was used to measure the iron uptake in this experiment since no accurate way is available to determine intrinsic iron uptake. Hence the values shown represent the minimum uptake that would have occurred in each digest, which is a similar assumption as made in human studies by Cook and Bjorn-Rasmussen.

## Claims

1. A composition for use in increasing absorption of iron when ingested by an animal or human, said composition comprising iron and peptides, wherein:
(a) the peptides are obtained from enzymatically hydrolyzed salmon backbones and heads;
(b) the composition is suitable for human or animal ingestion; and
(c) the peptides are greater than 50% of the composition.

2. The composition for use of claim 1, wherein the molecular weights of the peptides are 3500 Daltons or less.

3. The composition for use of claim 1 or 2, wherein the iron is greater than 0.01% of the composition.

4. The composition for use of any of claims 1 to 3, wherein said composition further comprises fish oil.

5. The composition for use of any of claims 1 to 4, wherein the composition is a dietary supplement or feed additive.

6. The composition for use of any of claims 1 to 5, wherein less than 50% of the peptides are chelated to the iron.

7. A composition of any one of claims 1-6 for use in treating an animal or human which has iron deficient anemia or is at risk for having iron deficient anemia and does not have a diet that is altered because of a disease or disorder.

## Patentansprüche

1. Zusammensetzung zur Verwendung bei der Erhöhung der Aufnahme von Eisen, wenn es von einem Tier oder einem Menschen eingenommen wird, wobei die Zusammensetzung Eisen und Peptide umfasst, wobei:
(a) die Peptide aus enzymatisch hydrolysiertem Lachs-Rückgrat und -Köpfen erhalten werden;
(b) die Zusammensetzung zur menschlichen oder tierischen Einnahme geeignet ist; und
(c) die Peptide mehr als 50% der Zusammensetzung betragen.

2. Zusammensetzung zur Verwendung nach Anspruch 1, wobei die Molekulargewichte der Peptide 3500 Dalton oder weniger betragen.

3. Zusammensetzung zur Verwendung nach Anspruch 1 oder 2, wobei das Eisen mehr als 0,01% der Zusammensetzung beträgt.

4. Zusammensetzung zur Verwendung nach einem der Ansprüche 1 bis 3, wobei die Zusammensetzung weiterhin Fischöl umfasst.

5. Zusammensetzung zur Verwendung nach einem der Ansprüche 1 bis 4, wobei die Zusammensetzung ein Nahrungsergänzungsmittel oder ein Futtermittelzusatz ist.

6. Zusammensetzung zur Verwendung nach einem der Ansprüche 1 bis 5, wobei weniger als 50% der Peptide chelatartig an das Eisen gebunden sind.

7. Zusammensetzung zur Verwendung nach einem der Ansprüche 1 bis 6 zur Verwendung bei der Behandlung eines Tiers oder eines Menschen, der Eisenmangelanämie hat oder bei dem das Risiko einer Eisenmangelanämie besteht und dessen Ernährung nicht auf Grund einer Krankheit oder Störung geändert ist.

## Revendications

1. Composition utilisable pour augmenter l'absorption de fer lorsqu'elle est ingérée par un animal ou un humain, ladite composition comprenant du fer et des peptides, dans laquelle :
(a) les peptides sont obtenus à partir d'épines dorsales et têtes enzymatiquement hydrolysés de saumon ;
(b) la composition est appropriée pour une ingestion par l'homme ou l'animal ; et
(c) les peptides représentent plus de 50 % de la composition.

2. Composition utilisable selon la revendication 1, dans laquelle les masses moléculaires des peptides sont de 3500 Daltons ou moins.

3. Composition utilisable selon la revendication 1 ou 2, dans laquelle le fer représente plus de 0,01 % de la composition.

4. Composition utilisable selon l'une quelconque des revendications 1 à 3, où ladite composition comprend en outre de l'huile de poisson.

5. Composition utilisable selon l'une quelconque des revendications 1 à 4, où la composition est un complément alimentaire ou un additif alimentaire.

6. Composition utilisable selon l'une quelconque des revendications 1 à 5, dans laquelle moins de 50 % des peptides sont chélatés au fer.

7. Composition selon l'une quelconque des revendications 1 à 6, destinée à être utilisée pour le traitement d'un animal ou d'un humain qui présente une anémie ferriprive ou est à risque de présenter une anémie ferriprive et n'a pas de régime qui est modifié à cause d'une maladie ou d'un trouble.
